# EUROPÄISCHE PATENTSCHRIFT

(11) **EP 2 410 960 B1**
(45) Veröffentlichungstag und Bekanntmachung des Hinweises auf die Patenterteilung: **11.03.2015**
(21) Anmeldenummer: 09776465.8
(22) Anmeldetag: 23.03.2009
(51) Int. Cl.: A61F 9/008

(54) **VORRICHTUNG FÜR DIE LASIK**
DEVICE FOR LASIK
DISPOSITIF POUR LASIK

(43) Veröffentlichungstag der Anmeldung: 01.02.2012
(73) Patentinhaber: WaveLight GmbH, 91058 Erlangen (DE)
(72) Erfinder: WÜLLNER, Christian, 91096 Möhrendorf (DE); VOGLER, Klaus, 90542 Eckental/Eschenau (DE); DONITZKY, Christof, 90542 Eschenau (DE)
(74) Vertreter: von Hellfeld, Axel
(86) Internationale Anmeldenummer: PCT/EP2009/002123
(87) Internationale Veröffentlichungsnummer: WO 2010/108501

(56) Entgegenhaltungen:
- EP-A- 1 792 593
- WO-A-99/34742
- WO-A-03/011175
- US-A1- 2007 027 439
- KRUEGER R R ET AL: "Rainbow Glare as an Optical Side Effect of IntraLASIK" OPHTHALMOLOGY, J. B. LIPPINCOTT CO., PHILADELPHIA, PA, US, Bd. 115, Nr. 7, 1. Juli 2008 (2008-07-01), Seiten 1187-1195.e1, XP022852665 ISSN: 0161-6420 [gefunden am 2007-12-27]

## Beschreibung

Die Erfindung betrifft eine Vorrichtung für die Laser in situ Keratomileusis (LASIK).

In der refraktiven ophthalmologischen Chirurgie werden durch Eingriffe am Auge eines Patienten die Brechungs- und Abbildungseigenschaften des Auges zur Korrektur oder Linderung von Sehfehlern verändert. Bekannt ist insbesondere das LASIK-Verfahren, bei dem die Hornhaut (Kornea) des Auges neu geformt wird. Beim herkömmlichen LASIK-Verfahren wird in einem ersten Schritt mit einem mechanischen Mikrokeratom ein flacher Hornhautschnitt gesetzt, um so einen sogenannten "Flap" (Deckel) zu erzeugen, der auf einer Seite fest mit der Hornhaut verbunden bleibt, so dass er aufgeklappt werden kann, um darunter liegendes Hornhautgewebe (Stroma) freizulegen. In dem freigelegten Stroma wird dann die Ablation, also die Entfernung von Gewebe mittels üblicherweise Excimerlaserstrahlung durchgeführt, woraufhin dann der Flap zurückgeklappt wird und wieder verheilt. Dabei bleibt das Epithel weitgehend unverletzt und der Heilungsprozess erfolgt relativ schnell und schmerzfrei. Bei einem herkömmlichen mechanischen Mikrokeratom oszilliert eine scharfe Klinge.

In jüngerer Zeit wird zum Schneiden des Flaps das mechanische Mikrokeratom zunehmend durch Laserstrahlung ersetzt. Die Laserstrahlung wird unterhalb der Oberfläche der Hornhaut fokussiert und auf einer Bahn geführt, wobei die Leistungsdichten so hoch sind, dass durch photodisruptive Effekte ein kontinuierlicher Schnitt entsteht. Um die hohen Leistungsdichten zu erhalten, werden kurze Laserpulse im Femtosekundenbereich eingesetzt, weshalb dieses Verfahren auch als Fs-LASIK bezeichnet wird.

Bei der genannten Fs-LASIK erfolgt also beim Schneiden des Flap eine dichte Folge von aneinandergereihten (Mikro-)Disruptionen aufgrund der hochgradig fokussierten Laserstrahlungspulse mit hoher Leistungsdichte. Insgesamt ergibt sich ein flächenhafter kontinuierlicher Schnitt im Stroma der Kornea. Über das verbleibende Scharnier (sogenanntes hinge) wird dann der Deckel (Flap) zur Seite geklappt und es erfolgt dann die eigentliche LASIK, also die Ablation (Entfernung) von Korneagewebe im offenen Stroma gemäß einem bestimmten Bearbeitungsprogramm zur Abtragung eines bestimmten Ablationsvolumens zur Neuformung der Kornea.

Die Erzeugung des Deckelchens bei der Fs-LASIK hat gegenüber dem Einsatz herkömmlicher mechanischer Mikrokeratome eine Reihe von Vorteilen und setzt sich deshalb zunehmend durch. Bei der Fs-LASIK (bisweilen auch als Fs-Mikrokeratom bezeichnet) können die Schnitttiefen mit sehr geringen Schwankungen genau in der gewünschten Weise eingehalten werden und es können auch besondere Randschnitte mit bestimmten Winkeln gebildet werden, die insbesondere Vorteile hinsichtlich der biomechanischen Stabilität des zurückgeklappten Deckelchens bringen.

Allerdings können bisweilen beim Fs-LASIK-Schnitt auch den Patienten störende Nebeneffekte auftreten in Form des sogenannten "rainbow glare effect" (Regenbogeneffekt). Dieser von einigen Patienten als störend empfundene Effekt besteht in der Wahrnehmung von farblichen Dispersionen bei Betrachtung bestimmter Strukturen und scharfer Kanten. Ursache dieses störenden Regenbogeneffektes ist die Erzeugung einer Art von Gitterstruktur in der durch die Photodisruption entstandenen Schnittfläche für die Erzeugung des Flaps. Die einzelnen Laser-Spots werden typischerweise so regelmäßig gesetzt, dass reguläre zwei-dimensionale Gitter mit Gitterkonstanten im µm-Bereich zumindest in bestimmten Bereichen des Schnittes entstehen können, die dann auch nach Abschluss der LASIK-Prozedur im verheilten Auge bestehen bleiben können und dann den bekannten Gittereffekt, also eine farblich aufgelöste Dispersion im oben beschriebenen Sinne verursachen können. Häufig bewirkt zwar die refraktive Ablation, also die gezielte Entfernung von Korneagewebe zur Neuformung der Kornea mit gewünschten Abbildungseigenschaften, auch eine Entfernung der genannten unerwünschten Gitterstrukturen, jedoch gelingt dies in der Regel nur in denjenigen Bereichen der Kornea, in denen relativ "viel" Gewebe abgetragen wird, während in denjenigen Korneabereichen, in denen die refraktive Ablation (also die Entfernung des gewünschten Ablationsvolumens zur Korrektur der Abbildungseigenschaften) nicht soviel Gewebe abträgt, häufig die unerwünschten Gitterstrukturen in der Kornea verbleiben, z.B. im zentralen Hornhautbereich bei der Hyperopiekorrektur.

In der EPA 1 977 725 wird diesem Problem dadurch begegnet, dass die Regularität der Spot-Positionen der Laserstrahlung beim Erzeugen des Schnittes soweit aufgehoben wird, dass die unerwünschte regelmäßige Gitterstruktur nicht entsteht. Dort wird ein stochastisches "Wobbeln" der die Strahlung steuernden Spiegel durchgeführt, um die unerwünschten regelmäßigen Gitterstrukturen bei Erzeugung des flap-Schnittes zu vermeiden. Allerdings muss trotz dieser stochastischen Schwankungen der Spot-Positionen gewährleistet sein, dass der Schnitt kontinuierlich durchgeht und eine hinreichend glatte Oberfläche im freigelegten Stroma gewährleistet ist. Das erfordert bei dem genannten bekannten Verfahren eine sehr aufwändige Optimierung und Steuerung.

Der Erfindung liegt die Aufgabe zugrunde, mit möglichst einfachen Mitteln bei der Fs-LASIK das Auftreten des sogenannten rainbow-glare-Effektes zu vermeiden.

Eine erfindungsgemäße Vorrichtung für Fs-LASIK ist ausgerüstet mit
- einer ersten Laserstrahlungsquelle zur Erzeugung von ersten Laserstrahlungspulsen mit einer Leistungsdichte zur Bewirkung von Disruption in Korneagewebe,
- ersten Mitteln zum Führen und Formen der ersten Laserstrahlungspulse in das Korneagewebe,
- einer zweiten Laserstrahlungsquelle zur Erzeugung von zweiten Laserstrahlungspulsen mit einer Leistungsdichte zur Bewirkung von Ablation von Korneagewebe,
- zweiten Mitteln zum Führen und Formen der zweiten Laserstrahlungspulse in Bezug auf die Kornea,
- einer Steuerung mit einem ersten Bearbeitungsprogramm zum Steuern der ersten Mittel und der ersten Laserstrahlurigspulse zur Erzeugung eines Schnittes in der Kornea derart, dass ein Deckel aufklappbar ist zur Freilegung einer Stromaoberfläche eines nicht zum Deckel gehörenden Stromabeltes, und mit
- einem zweiten Bearbeitungsprogramm , zum Steuern der zweiten Mittel und der zweiten Laserstrahlungspulse zur Neuformung und Änderung der Abbildungseigenschaften der Kornea zur Behandlung von Myopie oder Hyperopie, wobei
- das erste Bearbeitungsprogramm regelmäßige korneale Oberflächenstrukturen erzeugt, die einen Regenbogeneffekt bei den Abbildungseigenschaften der Kornea verursachen,
gekennzeichnet durch
- ein drittes Bearbeitungsprogramm, das eingerichtet ist, die zweiten Mittel und die zweiten Laserstrahlungspulse zur Entfernung der genannten regelmäßigen Strukturen auf der freigelegten Oberfläche des Stromabeltes zu steuern, wobei das dritte Bearbeitungsprogramm zusätzlich zu einem refraktiven Ablationsvolumen insbesondere im Randbereich der Kornea bei Myopie oder im mittleren Bereich der Kornea bei Hyperopie eine glattende Ablation bewirkt.

Bei der vorstehend beschriebenen Vorrichtung sind die genannten "regelmäßigen kornealen Oberflächenstrukturen", die einen Regenbogeneffekt bewirken, als diejenigen Strukturen zu verstehen, die beim Setzen des Schnittes für den Flap in unerwünschter Weise eine Gitterstruktur im oben beschriebenen Sinne erzeugen und deshalb erfindungsgemäß mit dem Bearbeitungsprogramm, welches anschließend im Stroma die ablative Formung durch Abtrag des sog. Ablationsvolumens bewirkt, gesondert entfernt oder zumindest so stark reduziert werden, dass der genannte Regenbogeneffekt verschwindet. In diesem Sinne ist das Ablationsvolumen als dasjenige Volumen der Kornea zu verstehen, welches für die refraktive Chirurgie vorab berechnet worden ist, um die gewünschte Abbildungsänderung des Auges insgesamt zu bewirken. Erfindungsgemäß ist darüber hinausgehend aber eine Glättung der nach Zurückklappen des Deckels freiliegenden Stromaoberfläche in denjenigen Bereichen, in denen unerwünschte Gitterstrukturen beim Flap-Schnitt entstanden sind, vorgesehen, wobei diese Glättung nicht wesentlich mit der Änderung der refraktiven Eigenschaften (Abbildungseigenschaften) des Auges zu tun hat.

Die Erfindung lässt sich auch so beschreiben, dass zusätzlich zur PRK, also der photorefraktiven Keratektomie (bei der durch Neuformung der Kornea deren Abbildungseigenschaften geändert werden), eine PTK vorgesehen ist, also eine phototherapeutische Keratektomie, bei der relative oberflächlich gelegene Defekte, Narben und andere Oberflächenstrukturen entfernt werden. Letzteres dient bei der Erfindung der Entfernung der genannten Gitterstrukturen auf der Oberfläche des freigelegten Stromas.

Diese Entfernung erfolgt bei der Photoablation in einem gesonderten Schritt, neben der refraktiven Neuformung der Kornea zur Änderung von deren Abbildungseigenschaften.

Der Patentanspruch, wie oben wiedergegeben, unterscheidet erste, zweite und dritte Bearbeitungsprogramme mit den jeweils angegebenen Funktionen. Diese Unterscheidung ist funktional zu verstehen, d.h. die drei genannten Funktionen der drei Bearbeitungsprogramme können in einem einzigen Computerprogramm kombiniert werden oder es können das zweite und das dritte Bearbeitungsprogramm, welche die ablativen Effekte bewirken, miteinander in einem einzigen Programm kombiniert werden.

Bei einer LASIK-Myopiebehandlung wird regelmäßig die Kornea abgeflacht, d.h. der Krümmungsradius der Kornea vergrößert. Dies bedeutet, dass das Ablationsvolumen hauptsächlich im mittleren Bereich der Kornea liegt, also um die optische Achse herum, während in äußeren Bereichen der Kornea kein oder nur wenig Gewebe abgetragen wird. Der Flap ist aber in der Regel über einen sehr weiten Bereich der Kornea geschnitten, so dass bei Behandlung von Myopie die durch den Flapschnitt erzeugten Gitterstrukturen in den Randbereichen des freigelegten Stromas durch den nachfolgenden Abtrag von Ablationsvolumen aus dem Stroma unter Umständen nicht völlig entfernt werden, wobei nach der Erfindung in diesen äußeren Bereichen der Kornea nahe dem Flap-Randschnitt die Gefahr des Verbleibens unerwünschter Gitterstrukturen im Korneagewebe besonders groß ist und deshalb nach der Erfindung zusätzlich zum Abtrag des refraktiven Ablationsvolumens auch in den Randbereichen der Kornea eine glättende Ablation erfolgt.

Andererseits wird bei der LASIK-Behandlung von Hyperopie das Ablationsvolumen regelmäßig so berechnet, dass der Krümmungsradius der Kornea verkleinert wird, also in den Randbereichen der Kornea, nahe dem Flap-Randschnitt, regelmäßig mehr Korneagewebe ablatiert wird als in zentralen, mittleren Bereichen der Kornea. Deshalb besteht die Gefahr, dass ohne die Erfindung bei der Hyperopie-Behandlung in mittleren Bereichen der Kornea beim Flap-Schnitt entstandene unerwünschte Gitterstrukturen verbleiben und so einen starken Regenbogeneffekt bewirken. Deshalb sieht die Erfindung bei Hyperopie-Behandlung vor, dass über das für die refraktive Korrektur der Kornea vorgesehene, randständige Ablationsvolumen hinaus auch in mittleren Bereichen der Kornea zusätzlich eine glättende Ablation zur Entfernung der Gitterstrukturen durchgeführt wird.

Nachfolgend werden Ausführungsbeispiele der Erfindung anhand der Zeichnung näher erläutert. Es zeigt:
Fig. 1 schematisch eine Vorrichtung zur Durchführung einer Fs-LASIK;
Fig. 2 schematisch einen Schnitt durch die Kornea eines Auges zur Erläuterung einer Myopie-Behandlung;
Fig. 3 einen schematischen Schnitt durch die Kornea eines Auges zur Erläuterung einer Hyperopie-Behandlung; und
Fig. 4 einen Schnitt entsprechend Fig. 3 einschließlich der erfindungsgemäß angestrebten Zieloberfläche.

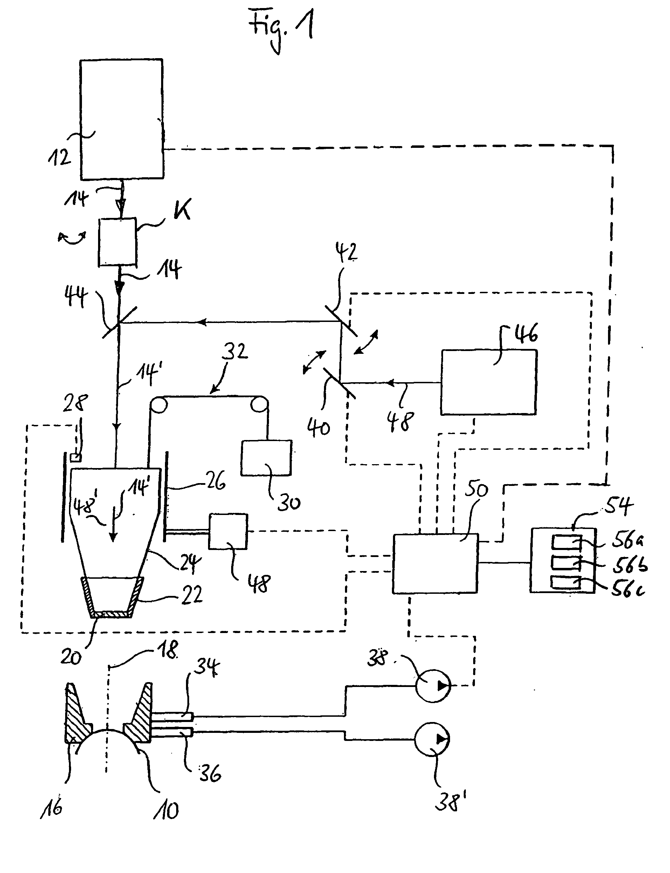

Fig. 1 zeigt eine Vorrichtung für die Fs-LASIK, wobei üblicherweise zwei unterschiedliche Laserstrahlungsquellen eingesetzt werden, nämlich eine erste Laserstrahlungsquelle zur Erzeugung von Femtosekundenpulsen für die Durchführung des Flap-Schnittes durch Photodisruption und eine zweite Laserstrahlungsquelle zur Erzeugung einer anderen Art von Laserstrahlungspulsen mit einer geringeren Leistungsdichte zur Durchführung der Ablation von Korneagewebe. Üblicherweise sind im Stand der Technik hierfür zwei verschiedene Laserstrahlungssysteme mit getrennten optischen Systemen zur Strahlformung und Strahlführung in Bezug auf das Auge vorgesehen, die wahlweise (unabhängig voneinander) in Bezug auf das zu behandelnde Auge ausgerichtet werden. In Fig. 1 sind jedoch die beiden Systeme quasi zusammengefasst der Einfachheit halber dargestellt.

Eine erste Laserstrahlungsquelle 12 dient zur Erzeugung von als solches in dieser Technik bekannten Femtosekundenpulsen 14, die eine so hohe Leistungsdichte haben, dass sie nach Fokussierung im Inneren der Kornea dort eine disruptiven Effekt bewirken. Die Mittel zum Formen und Führen dieser ersten Laserstrahlungspulse 14 sind in Fig. 1 mit dem Bezugszeichen K zusammenfassend angedeutet und als solche im Stand der Technik bekannt. Über einen für die ersten Laserstrahlungspulse 14 durchlässigen Spiegel 44 wird diese Laserstrahlung in Richtung auf das Auge 10 geführt. Das Auge 10 ist mittels eines Saugringes 16 fixiert und eine Applanationslinse 20 wird koaxial auf die Achse 18 des Saugrings 16 bezogen in der Figur nach unten abgesenkt, so dass eine Schnittstelleneinheit 22 in eine konische Aufnahme am Saugring 16 eingreift. Mittels einer Fokussieroptik 24 werden die ersten Laserstrahlungspulse 14' zur Erzeugung des Flap-Schnittes in an sich bekannter Weise in eine vorab berechnete Fläche unterhalb der Oberfläche der Kornea des Auges 10 fokussiert. Die Fokussieroptik 24 wird in einer Halterung 26 geführt. Die Führung erfolgt mittels eines Ortssensors 28, wobei die Fokussieroptik 24 über ein Gegengewicht 30 und eine Wippe frei schwebend aufgehängt ist, um eine das Auge so gut wie nicht belastende Ankoppelung der Schnittstelleneinheit 22 an das Auge 10 zu ermöglichen. Der Saugring 16 wird mittels als solches bekannter Anschlussstutzen 34, 36 und Vakuumpumpen 38 fixiert. Die vorstehend beschriebene Fokussieroptik 24 dient hauptsächlich für die Fokussierung der nachfolgend beschriebenen zweiten Laserstrahlungspulse für die Ablation. Für die ersten Laserstrahlungspulse 14 sind optische Formungs- und Führungsmittel für die Strahlung vorgesehen, die als solches im Stand der Technik bekannt sind und in Fig. 1 mit dem Funktionsblock "K" angedeutet sind, wodurch dann auch die Steuerung der Fokusse der ersten Laserstrahlungspulse in Raum und Zeit in bekannter Weise erfolgt.

Eine zweite Laserstrahlungsquelle 46 dient zur Erzeugung zweiter Laserstrahlungspulse 48 für die Ablation. Diese zweiten Laserstrahlungspulse 48 werden über als solches bekannte Spiegel (inklusive Scannerspiegel) 40, 42, 44 in die Fokussieroptik 24 gerichtet. Einzelheiten dieser Anordnung sind in der internationalen Patentanmeldung PCT/EP2008/006962 näher erläutert, die hier durch Bezugnahme voll eingeschlossen ist.

Eine Computersteuerung 50 steuert sämtliche steuerbaren Komponenten des Systems, wobei in Fig. 1 die Steuerverbindungen durch gestrichelte Linien angezeigt sind.

In einem Speicher 54 sind insbesondere ein erstes Bearbeitungsprogramm 56a, ein zweites Bearbeitungsprogramm 56b, und ein drittes Bearbeitungsprogramm 56c abgelegt, auf die die Steuerung 50 wahlweise zugreifen kann. Diese drei Bearbeitungsprogramme werden nachfolgend näher beschrieben.

Mit dem ersten Bearbeitungsprogramm 56a steuert die Computersteuerung 50 den Laser 12 und die damit erzeugten ersten Laserstrahlungspulse 14 zur Durchführung des beschriebenen Flap-Schnittes mittels Photodisruption. Dies ist als solches im Stand der Technik bekannt und auch das Phänomen, dass dabei auf der Oberfläche des nach Zurückklappen des Flaps freiliegenden Stromas unerwünschte Gitterstrukturen im obigen Sinn erzeugt werden.

Zur anschließenden Ablation von Korneagewebe zur Durchführung der PRK, greift die Computersteuerung 50 auf das zweite Bearbeitungsprogramm 56b, so dass ein im Voraus in bekannter Weise berechnetes Ablationsvolumen aus dem Stroma der Kornea ablatiert wird, um in gewünschter Weise die Abbildungseigenschaften der Kornea abzuändern.

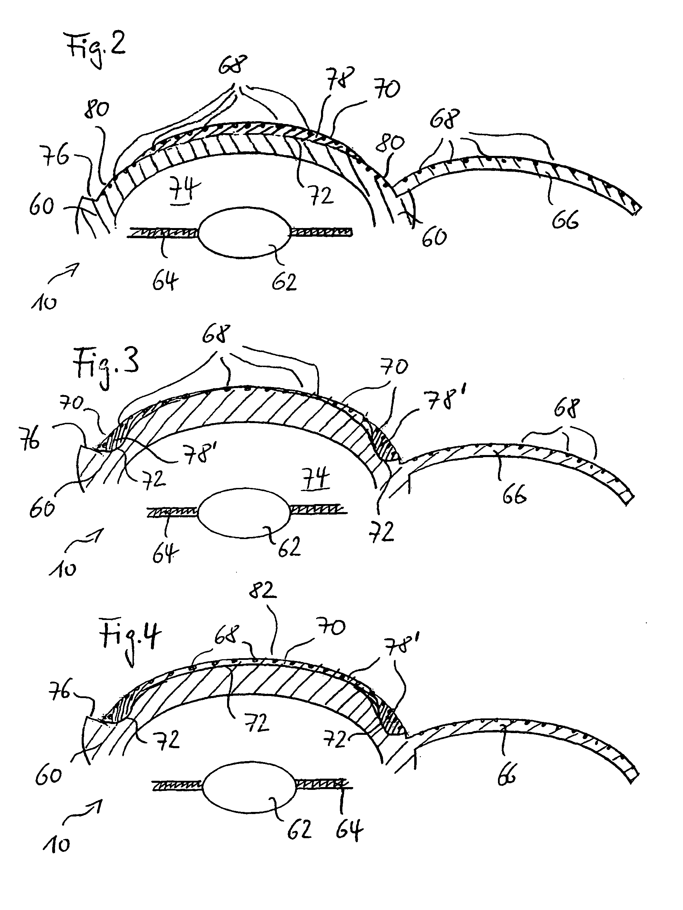

Die bei Abarbeitung des ersten Bearbeitungsprogramms 56a möglicherweise erzeugten unerwünschten Gitterstrukturen werden sodann in einem dritten Schritt gemäß einem dritten Bearbeitungsprogramm 56c (PTK) entfernt. Dies ist anhand der Fig. 2 bis 4 näher beschrieben.

Die Fig. 2 bis 4 zeigen die Kornea 60 eines Auges 10 schematisch im Schnitt. Dargestellt sind nur die hier näher interessierenden Teile des Auges (die Retina etc. sind also weggelassen).

Gezeigt ist neben der Kornea 60 noch die Augenlinse 62 und die Iris 64.

Mit dem ersten Bearbeitungsprogramm 56a wird mittels der ersten Laserstrahlungsquelle 12 in an sich bekannter Weise der Flap-Schnitt der Fs-LASIK durchgeführt. Dabei entstehen die oben erläuterten, unerwünschten Gitterstrukturen, die in den Figuren mit dem Bezugszeichen 68 schematisch angedeutet sind, also Vertiefungen in der jeweils freiliegenden Oberfläche des Korneagewebes mit den beschriebenen unerwünschten Wirkungen aufgrund ihrer regelmäßigen Struktur. Diese gitterartige Struktur hat typischerweise eine Gitterkonstante im µm-Bereich mit den oben erläuterten Folgen hinsichtlich des "rainbow glare". Das Bezugszeichen 68 deutet also ein sog. Mikrogitter an.

In den Figuren ist die Ausgangsoberfläche des nach Zurückklappen des Flaps 66 freiliegenden Stromas mit 70 bezeichnet. Gemäß Fig. 2 ist also das aus den Vertiefungen 68 gebildete Mirkogitter über die gesamte freiliegende Fläche des Stromas der Kornea verteilt. Beim Ausführungsbeispiel gemäß Fig. 2 soll Myopie behandelt werden, also der Krümmungsradius der Kornea nach der Behandlung vergrößert sein, die Kornea also abgeflacht werden. Das ist in Fig. 2 durch das Ablationsvolumen 78 (eng schraffiert) dargestellt, also dasjenige Volumen der Kornea, welches durch Ablation mittels der zweiten Laserstrahlungspulse 48 abgetragen werden soll. Die dabei angestrebte Zieloberfläche ist mit dem Bezugszeichen 72 versehen. Somit ist das Ablationsvolumen 78 in Fig. 2 der eng schraffierte Bereich zwischen der Ausgangsoberfläche 70 und der Zieloberfläche 72. Bei Myopie-Behandlung verschwinden deshalb im mittleren Bereich der Kornea die das Mikrogitter bildenden Vertiefungen 68 quasi von selbst, da hinreichend Korneagewebe abgetragen wird, um am Schluss die unerwünschte Gitterstruktur weitgehend zum Verschwinden zu bringen. Deshalb ist nicht unbedingt erforderlich, in diesem mittleren Bereich der Kornea bei einer typischen Myopiebehandlung gesonderte Maßnahmen zur Glättung der Oberfläche und zum Entfernen der unerwünschten Gitterstruktur vorzusehen (obwohl dies nach der Erfindung nicht ausgeschlossen ist). Allerdings bleibt, wie Fig. 2 zeigt, in Randbereichen 80 des freiliegenden Stromabettes die durch die Vertiefungen 68 gebildete unerwünschte Mikrostruktur auch nach Durchführung der Ablation weitgehend erhalten, so dass in Randbereichen 80 der Kornea, also nahe dem Flap-Randschnitt 76, besondere Maßnahmen zur Entfernung des durch die Vertiefungen 68 erzeugten Mikrogitters erforderlich sind. Hierzu greift die Computersteuerung 50 auf das dritte Bearbeitungsprogramm 56c zurück, welches die zweiten Laserstrahlungspulse 48 so über die freiliegende Oberfläche des Stromas führt, dass die Oberfläche auch in den Randbereichen 80 geglättet wird. Hierfür können als solches bekannte Verfahren der phototherapeutischen Keratektomie (PTK) herangezogen werden, siehe zum Beispiel A. N. Kollias, et al., Journal of Refractive Surgery, vol. 23, September 2007, S. 703-708, oder Arch. Ophthalmology, vol. 109, June 1991, S. 860-863 oder P. Vinciguerra, F. Camesasca, Journal of refractive Surgery, vol. 20, 2004, S. 555-563. Hierzu kann auf die zitierten bekannten Verfahren der PTK zurückgegriffen werden.

In Abwandlung der vorstehend beschriebenen Ausführungsbeispiele kann diese Glättung der nach Zurückklappen des Flaps freiliegenden Stromaoberfläche auch nicht durch das dritte Bearbeitungsprogramm mit Laserstrahlung durchgeführt werden, sondern durch andere PTK-Techniken, zum Beispiel durch eine manuelles Glätten zum Beispiel der Randbereiche 80 beim Ausführungsbeispiel nach Fig. 2 mit Auftragung geeigneter Flüssigkeiten (siehe die zitierte Literatur) und zum Beispiel einer "brush". Bei dieser Variante die nicht Teil der Erfindung ist wird also das unerwünschte Mikrogitter mechanisch (ohne Laserstrahlung) "weggeschliffen".

Nach Entfernung der unerwünschten Gitterstruktur und Zurückklappen des Flaps 66 wird regelmäßig erreicht, dass keine unerwünschte Gitterstruktur letztlich im refraktiv abgeänderten Komea-Gewebe verbleibt. Eventuell auf der Innenseite des Flaps verbleibende Mikrostrukturen reichen nicht aus, um die unerwünschte Gitterstruktur zu bilden oder kommen nach Zurückklappen des Flaps nicht haargenau über den ursprünglichen Gitterstrukturen zu liegen, so dass die vorstehend beschriebenen Glättungsmaßnahmen bezüglich der Stromaoberfläche ausreichen. Es hat sich experimentell herausgestellt, dass eventuell im Flap verbleibende Gitterstrukturen weniger kritisch sind als die vorstehend beschriebenen Gitterstrukturen im Stroma. Dies erklärt sich daraus, dass bei der Photodisruption die Fs-Pulse in Richtung der Strahlungsausbreitung einen relativ scharfen Einsatz (Start) haben. Beim LIOB (laser induced optical breakdown) sind deshalb die im Flap verbleibenden Vertiefungen deutlich weniger ausgeprägt (tief) als im Stromabett. Die Vertiefungen im Flap sind typischerweise weniger tief als 5 µm, während die Vertiefungen im Stromabett deutlich tiefer sind und fast die Rayleighlänge (15 bis 20 µm) erreichen. Alternativ können die unerwünschten Gitterstrukturen 68 im Flap 66 auch in der vorstehend beschriebenen Weise mechanisch entfernt werden.

Fig. 3 zeigt die Behandlung einer Hyperopie mit Fs-LASIK, wobei in allen Figuren einander entsprechende Teile mit gleichen Bezugszeichen versehen sind. Wie oben bereits erläutert ist, liegt bei der Hyperopie-Behandlung das Ablationsvolumen 78' hauptsächlich in Randbereichen des freigelegten Stromas, also nahe am Flap-Randschnitt 76, was in Fig. 3 durch die eng schraffierten Bereiche dargestellt ist, welche das Ablationsvolumen 78' markieren. Durch Entfernung des Ablationsvolumens 78' mittels der zweiten Laserstrahlungspulse 48 verschwinden auch in diesen Bereichen die unerwünschten Gitterstrukturen 68, wohingegen diese Strukturen im mittleren Bereich weitgehend in der Ausgangsoberfläche 70 erhalten bleiben, wie Fig. 3 darstellt. Deshalb wird bei der Hyperopie-Behandlung gemäß Fig. 4 auch im mittleren Bereich 82 des nach Zurückklappen des Flaps 66 freiliegenden Stromas eine Glättung dahingehend durchgeführt, dass die Mikrogitter 68 verschwinden und eine glatte Zieloberfläche 72 erreicht wird. Hierfür dienen die oben näher erläuterten PTK-Techniken, also entweder das dritte Bearbeitungsprogramm 56c oder auch andere PTK-Glättungstechniken gemäß der vorstehend genannten Literatur.

Wird das dritte Bearbeitungsprogramm 56c eingesetzt, dann wird insbesondere im mittleren Bereich des freiliegenden Stromas eine Schicht zwischen der Ausgangsoberfläche 70 und der Zieloberfläche 72 abgetragen mit einer Dicke von bis zu 10 µm, was über die Computersteuerung 50 mit dem Excimer-Laser 46 gut durchführbar ist. Ein Flap 66 ist typischerweise 100-160 µm dick.

Dabei kann die der Glättung dienende Ablation mit dem dritten Bearbeitungsprogramm im zweiten Bearbeitungsprogramm, welches die refraktive Korrektur der Kornea bewirkt, berücksichtigt werden, d.h. bei Berechnung des Ablationsvolumens und dementsprechend der Erzeugung des zweiten Bearbeitungsprogramms für die Photoablation kann von vornherein berücksichtigt werden, dass über die gesamte Kornea-Oberfläche oder ausgewählte Teile der Kornea-Oberfläche (bei Fig. 4 also der mittlere Bereich 82) ein gleichmäßiger Gewebeabtrag erfolgt. Analoges gilt für die Myopie-Behandlung gemäß Fig. 2, bei der über die gesamte freiliegende Oberfläche des Stroma oder auch Teile davon (wie insbesondere die Randbereiche 80 gemäß Fig. 2) ein glättender Gewebeabtrag erfolgt, der im zweiten Bearbeitungsprogramm für die Rechnung der refraktiven Wirkung berücksichtigt ist.

## Patentansprüche

1. Vorrichtung für Femtosekunden-Laser in situ Keratomileusis (fs-LASIK) mit
- einer ersten Laserstrahlungsquelle (12) zur Erzeugung von ersten Laserstrahlungspulsen (14) mit einer Leistungsdichte zur Bewirkung von Disruptionen in Korneagewebe,
- ersten Mitteln (24, 44, K) zum Führen und Formen der ersten Laserstrahlungspulse (14) in das Korneagewebe,
- einer zweiten Laserstrahlungsquelle (46) zur Erzeugung von zweiten Laserstrahlungspulsen (48) mit einer Leistungsdichte zur Bewirkung von Ablation von Korneagewebe,
- zweiten Mitteln (40, 42, 44, 24) zum Führen und Formen der zweiten Laserstrahlungspulse (48) in Bezug auf die Kornea,
- einer Steuerung (50) mit einem ersten Bearbeitungsprogramm (56a) zum Steuern der ersten Mittel und der ersten Laserstrahlungspulse (14) zur Erzeugung eines Schnittes in der Kornea derart, dass ein Deckel (66) aufklappbar ist zur Freilegung einer Stromaoberfläche (70) eines nicht zum Deckel (66) gehörenden Stromabettes, und mit
- einem zweiten Bearbeitungsprogramm (56b), zum Steuern der zweiten Mittel und der zweiten Laserstrahlungspulse (48) zur Neuformung und Änderung der Abbildungseigenschaften der Kornea zur Behandlung von Myopie wobei
- das erste Bearbeitungsprogramm (56a) regelmäßige korneale Oberflächenstrukturen (68) auf der Oberfläche (70) des genannten Stromabettes erzeugt, die einen Regenbogeneffekt bei den Abbildungseigenschaften der Kornea verursachen,
**gekennzeichnet durch**
- ein drittes Bearbeitungsprogramm (56c), das eingerichtet ist ,die zweiten Mittel (40, 42, 44, 24) und die zweiten Laserstrahlungspulse (48) zur Entfernung der genannten regelmäßigen Strukturen (68) auf der freigelegten Oberfläche (70) des Stromabettes zu steuern, wobei das dritte Bearbeitungsprogramm (56c) zusätzlich zu einem refraktiven Ablationsvolumen (78) insbesondere im Randbereich (80) der Kornea (60) eine glättende Ablation bewirkt.

2. Vorrichtung für Femtosekunden-Laser in situ Keratomileusis (fs-LASIK) mit
- einer ersten Laserstrahlungsquelle (12) zur Erzeugung von ersten Laserstrahlungspulsen (14) mit einer Leistungsdichte zur Bewirkung von Disruptionen in Korneagewebe,
- ersten Mitteln (24, 44, K) zum Führen und Formen der ersten Laserstrahlungspulse (14) in das Korneagewebe,
- einer zweiten Laserstrahlungsquelle (46) zur Erzeugung von zweiten Laserstrahlungspulsen (48) mit einer Leistungsdichte zur Bewirkung von Ablation von Korneagewebe,
- zweiten Mitteln (40, 42, 44, 24) zum Führen und Formen der zweiten Laserstrahlungspulse (48) in Bezug auf die Kornea,
- einer Steuerung (50) mit einem ersten Bearbeitungsprogramm (56a) zum Steuern der ersten Mittel und der ersten Laserstrahlungspulse (14) zur Erzeugung eines Schnittes in der Kornea derart, dass ein Deckel (66) aufklappbar ist zur Freilegung einer Stromaoberfläche (70) eines nicht zum Deckel (66) gehörenden Stromabettes, und mit
- einem zweiten Bearbeitungsprogramm (56b), zum Steuern der zweiten Mittel und der zweiten Laserstrahlungspulse (48) zur Neuformung und Änderung der Abbildungseigenschaften der Kornea zur Behandlung von Hyperopie, wobei
- das erste Bearbeitungsprogramm (56a) regelmäßige korneale Oberflächenstrukturen (68) auf der Oberfläche (70) des genannten Stromabettes erzeugt, die einen Regenbogeneffekt bei den Abbildungseigenschaften der Kornea verursachen,
**gekennzeichnet durch**
- ein drittes Bearbeitungsprogramm (56c), das eingerichtet ist ,die zweiten Mittel (40, 42, 44, 24) und die zweiten Laserstrahlungspulse (48) zur Entfernung der genannten regelmäßigen Strukturen (68) auf der freigelegten Oberfläche (70) des Stromabettes zu steuern, wobei das dritte Bearbeitungsprogramm (56c) zusätzlich zu einem refraktiven Ablationsvolumen (78') insbesondere im mittleren Bereich (82) der Kornea (60) eine glättende Ablation bewirkt.

## Claims

1. Apparatus for laser femtosecond in-situ keratomileusis (fs-LASIK), with:
- a first laser radiation source (12) for generating first laser radiation pulses (14) having a power density for bringing about disruptions in corneal tissue,
- first means (24, 44, K) for guiding and shaping the first laser radiation pulses (14) into the corneal tissue,
- a second laser radiation source (46) for generating second laser radiation pulses (48) having a power density for bringing about ablation of corneal tissue,
- second means (40, 42, 44, 24) for guiding and shaping the second laser radiation pulses (48) in relation to the cornea,
- a controller (50) with a first treatment program (56a) for controlling the first means and the first laser radiation pulses (14) for generating an incision in the cornea (60) such that a flap (66) can be opened to expose a stromal surface (70) of a stromal bed not being part of the flap (66), and with
- a second treatment program (56b) for controlling the second means and the second laser radiation pulses (48) for reshaping the cornea and changing its imaging properties for treatment of myopia, wherein
- the first treatment program (56a) produces regular corneal surface structures (68) which cause a rainbow-glare effect in connection with the imaging properties of the cornea,
**characterized by**
- a third treatment program (56c) which controls the second means (40, 42, 44, 24) and the second laser radiation pulses (48) for removing the aforementioned regular structures (68) at said exposed surface (70) of the stromal bed, wherein the third treatment program (56c) brings about, in addition to a refractive ablation volume (78'), a smoothing ablation particularly in the marginal region (80) of the cornea (60).

2. Apparatus for laser femtosecond in-situ keratomileusis (fs-LASIK), with:
- a first laser radiation source (12) for generating first laser radiation pulses (14) having a power density for bringing about disruptions in corneal tissue,
- first means (24, 44, K) for guiding and shaping the first laser radiation pulses (14) into the corneal tissue,
- a second laser radiation source (46) for generating second laser radiation pulses (48) having a power density for bringing about ablation of corneal tissue,
- second means (40, 42, 44, 24) for guiding and shaping the second laser radiation pulses (48) in relation to the cornea,
- a controller (50) with a first treatment program (56a) for controlling the first means and the first laser radiation pulses (14) for generating an incision in the cornea (60) such that a flap (66) can be opened to expose a stromal surface (70) of a stromal bed not being part of the flap (66), and with
- a second treatment program (56b) for controlling the second means and the second laser radiation pulses (48) for reshaping the cornea and changing its imaging properties for treatment of hyperopia, wherein
- the first treatment program (56a) produces regular corneal surface structures (68) which cause a rainbow-glare effect in connection with the imaging properties of the cornea,
**characterized by**
- a third treatment program (56c) which controls the second means (40, 42, 44, 24) and the second laser radiation pulses (48) for removing the aforementioned regular structures (68) at said exposed surface (70) of the stromal bed, wherein the third treatment program (56c) brings about, in addition to a refractive ablation volume (78'), a smoothing ablation particularly in the middle region (82) of the cornea (60).

## Revendications

1. Dispositif pour kératomileusis par laser femtoseconde in situ (LASIK femtoseconde), comprenant :
- une première source de rayonnement laser (12) pour générer des premières impulsions de rayonnement laser (14) dont la densité de puissance permet d'obtenir des disruptions dans le tissu cornéen,
- des premiers moyens (24, 44, K) pour guider et former les premières impulsions de rayonnement laser (14) dans le tissu cornéen,
- une deuxième source de rayonnement laser (46) pour générer des deuxièmes impulsions de rayonnement laser (48) dont la densité de puissance permet d'obtenir l'ablation de tissu cornéen,
- des deuxièmes moyens (40, 42, 44, 24) pour guider et former les deuxièmes impulsions de rayonnement laser (48) par rapport à la cornée,
- une commande (50) dotée d'un premier programme de traitement (56a) pour commander les premiers moyens et les premières impulsions de rayonnement laser (14) pour effectuer une découpe dans la cornée de manière à réaliser un capot (66) réclinable pour mettre à nu une surface stromale (70) d'un lit stromal qui ne fait pas partie intégrante du capot (66), et
- un deuxième programme de traitement (56b) pour commander les deuxièmes moyens et les deuxièmes impulsions de rayonnement laser (48) en vue de reformer et de modifier les propriétés fonctionnelles de la cornée pour corriger la vision des myopes,
- le premier programme de traitement (56a) formant à la surface (70) dudit lit stromal des structures de surface cornéennes régulières (68) qui produisent un effet d'arc-en-ciel au niveau des propriétés fonctionnelles de la cornée,
**caractérisé par**
- un troisième programme de traitement (56c), qui est conçu pour commander les deuxièmes moyens (40, 42, 44, 24) et les deuxièmes impulsions de rayonnement laser (48) en vue d'éliminer lesdites structures régulières (68) sur la surface (70) mise à nu du lit stromal, le troisième programme de traitement (56c) permettant d'obtenir, en plus d'un volume d'ablation (78) réfractif, une ablation lissante, en particulier dans la zone périphérique (80) de la cornée (60).

2. Dispositif pour kératomileusis par laser femtoseconde in situ (LASIK femtoseconde), comprenant :
- une première source de rayonnement laser (12) pour générer des premières impulsions de rayonnement laser (14) dont la densité de puissance permet d'obtenir des disruptions dans le tissu cornéen,
- des premiers moyens (24, 44, K) pour guider et former les premières impulsions de rayonnement laser (14) dans le tissu cornéen,
- une deuxième source de rayonnement laser (46) pour générer des deuxièmes impulsions de rayonnement laser (48) dont la densité de puissance permet d'obtenir l'ablation de tissu cornéen,
- des deuxièmes moyens (40, 42, 44, 24) pour guider et former les deuxièmes impulsions de rayonnement laser (48) par rapport à la cornée,
- une commande (50) dotée d'un premier programme de traitement (56a) pour commander les premiers moyens et les premières impulsions de rayonnement laser (14) pour effectuer une découpe dans la cornée de manière à réaliser un capot (66) réclinable pour mettre à nu une surface stromale (70) d'un lit stromal qui ne fait pas partie intégrante du capot (66), et
- un deuxième programme de traitement (56b) pour commander les deuxièmes moyens et les deuxièmes impulsions de rayonnement laser (48) en vue de reformer et de modifier les propriétés fonctionnelles de la cornée pour corriger la vision des hypermétropes,
- le premier programme de traitement (56a) formant à la surface (70) dudit lit stromal des structures de surface cornéennes régulières (68) qui produisent un effet d'arc-en-ciel au niveau des propriétés fonctionnelles de la cornée,
**caractérisé par**
- un troisième programme de traitement (56c), qui est conçu pour commander les deuxièmes moyens (40, 42, 44, 24) et les deuxièmes impulsions de rayonnement laser (48) en vue d'éliminer lesdites structures régulières (68) sur la surface (70) mise à nu du lit stromal, le troisième programme de traitement (56c) permettant d'obtenir, en plus d'un volume d'ablation (78) réfractif, une ablation lissante, en particulier dans la zone médiane de la cornée.
